# FASCICULE DE BREVET EUROPEEN

(11) **EP 4 143 567 B1**
(45) Date de publication et mention de la délivrance du brevet: **08.05.2024**
(21) Numéro de dépôt: 21716173.6
(22) Date de dépôt: 31.03.2021
(51) Int. Cl.: G01N 33/24, G06F 18/2413, G06T 7/11, G06T 7/194, G06V 10/20, G06V 10/26, G06N 3/045, G06N 20/10

(54) **PROCEDE DE DETECTION D'AU MOINS UN CONSTITUANT GEOLOGIQUE D'UN ECHANTILLON DE ROCHE**
VERFAHREN ZUR DETEKTION MINDESTENS EINES GEOLOGISCHEN BESTANDTEILS EINER GESTEINSPROBE
METHOD FOR DETECTING AT LEAST ONE GEOLOGICAL CONSTITUENT OF A ROCK SAMPLE

(30) Priorité: 27.04.2020 FR 2004169
(43) Date de publication de la demande: 08.03.2023
(73) Titulaire: IFP Energies nouvelles, 92500 Rueil-Malmaison (FR)
(72) Inventeur: DESROZIERS, Sylvain, 92852 RUEIL-MALMAISON CEDEX (FR); FERAILLE, Mathieu, 92852 RUEIL-MALMAISON CEDEX (FR); CLOCHARD, Vincent, 92852 RUEIL-MALMAISON CEDEX (FR); BOUZIAT, Antoine, 92852 RUEIL-MALMAISON CEDEX (FR); HAMON, Youri, 92852 RUEIL-MALMAISON CEDEX (FR); MOREAUD, Maxime, 92852 RUEIL-MALMAISON CEDEX (FR)
(74) Mandataire: IFP Energies nouvelles
(86) Numéro de dépôt international: PCT/EP2021/058411
(87) Numéro de publication internationale: WO 2021/219317

(56) Documents cités:
- CHENG SU ET AL: "Rock Classification in Petrographic Thin Section Images Based on Concatenated Convolutional Neural Networks", ARXIV.ORG, CORNELL UNIVERSITY LIBRARY, 201 OLIN LIBRARY CORNELL UNIVERSITY ITHACA, NY 14853, 23 mars 2020 (2020-03-23), XP081627653,
- WEI REN ET AL: "Identifying Rock Thin Section Based on Convolutional Neural Networks", PROCEEDINGS OF 2019 THE 9TH INTERNATIONAL WORKSHOP ON COMPUTER SCIENCE AND ENGINEERING, 2 septembre 2019 (2019-09-02), pages 345-351, XP055772970, DOI: 10.18178/wcse.2019.06.052 ISBN: 978-981-1416-84-2
- PIRES DE LIMA RAFAEL ET AL: "Deep convolutional neural networks as a geological image classification tool", THE SEDIMENTARY RECORD, vol. 17, no. 2, 30 juin 2019 (2019-06-30), pages 4-9, XP055772993, ISSN: 1543-8740, DOI: 10.2110/sedred.2019.2.4
- BOUREL BENJAMIN ET AL: "Automated recognition by multiple convolutional neural networks of modern, fossil, intact and damaged pollen grains", COMPUTERS & GEOSCIENCES, PERGAMON, AMSTERDAM, NL, vol. 140, 14 avril 2020 (2020-04-14), XP086167071, ISSN: 0098-3004, DOI: 10.1016/J.CAGEO.2020.104498 [extrait le 2020-04-14]

## Description

### Domaine technique

La présente invention concerne le domaine de la détection de constituants géologiques d'un échantillon de roche.

La caractérisation d'un échantillon rocheux nécessite souvent de repérer et de compter certains constituants spécifiques, présents à l'intérieur de l'échantillon, comme par exemple des microfossiles, des nanofossiles, des débris végétaux, des minéraux, des spores de pollen, ou analogues. Pour un spécialiste, ce travail peut notamment être réalisé en observant directement l'échantillon rocheux à l'oeil nu, ou en découpant une fine tranche, qu'on appelle « lame mince », pour l'observer au microscope optique. Cela permet de catégoriser plus précisément la roche et d'estimer ses propriétés physiques, pour des applications en génie civil, pour la dépollution des sites, pour des recherches de ressources minérales ou énergétiques souterraines, etc.

De plus, dans le cas où ces constituants géologiques sont des microfossiles (micro-paléontologie) ou des éléments végétaux (palynologie) dans des roches sédimentaires, ce type d'analyse permet d'estimer à la fois l'âge, l'environnement (marin, côtier, continental...) et le contexte climatique au moment du dépôt du sédiment. Appliqué sur plusieurs échantillons de la même succession sédimentaire, c'est alors un moyen de reconstituer l'évolution du climat dans une zone géographique à travers les âges géologiques (paléo-climatologie).

### Technique antérieure

La détection et le comptage de certains constituants géologiques est traditionnellement réalisé manuellement par un expert humain, ce qui nécessite une formation adéquate et est souvent très fastidieux.

Pour ce qui est des microfossiles, on peut consulter, notamment le Manuel de micro-paléontologie publié en 2011 par Mathieu, Bellier et Granier (ISBN 978-2-916733-04-3), ainsi que la base de données des foraminifères sur le site http://www.marinespecies.org/foraminifera/index.php. Une telle base de données comporte un très grand nombre de données. Cette base n'est donc exploitable que par un expert, et pas dans un procédé automatisé.

Pour ce qui est des minéraux, un ouvrage comparable est l'atlas de pétrographie publié en 2001 par McKenzie et Guilford chez Dunod (ISBN 978-2-100054-59-6).

En ce qui concerne, les pollens, on peut consulter l'ouvrage de Paleopalynology de Alfred Traverse publié en 2007 (ISBN 978-1-4020-5610-9).

Afin de rendre ces analyses à la fois plus accessibles et plus rapides, l'automatisation de la détection et du comptage par des méthodes numériques a été considérée. Cela peut être réalisé par des systèmes experts, des techniques de seuillage plus ou moins avancées, diverses méthodes d'analyse et de traitement d'images, et surtout par des méthodes d'apprentissage automatique, notamment basées sur des réseaux de neurones artificiels. Pour les microfossiles, on peut se référer notamment à : Beaufort, L., & Dollfus, D. (2004). Automatic recognition of coccoliths by dynamical neural networks. Marine Micropaleontology, 51(1-2), 57-73. Cette méthode est également décrite dans la demande de brevet WO2015132531A1. Toutefois, une telle méthode nécessite un grand nombre d'images d'apprentissage pour la méthode d'apprentissage d'automatique. La demande de brevet cite notamment l'utilisation de 10000 images obtenues par microscopie, et 100 images par groupe morphologique. Ce grand nombre d'images d'apprentissage nécessite un temps très important de préparation et un nombre d'opérations préparatoires très important : il faut préparer les lames minces (notamment par découpe de la roche, imprégnation, et rodage) et réaliser les observations au moyen du microscope pour chaque lame mince. Ensuite, pour chaque image, il faut manuellement repérer les constituants considérés, ce qui nécessite un travail conséquent pour un expert.

En ce qui concerne une méthode d'apprentissage automatique appliquée aux minéraux, on peut se référer au document : Thompson, S., Fueten, F., & Bockus, D. [2001] Mineral identification using artificial neural networks and the rotating polarizer stage. Computers & Geosciences, 27(9), 1081-1089. De la même manière, cette méthode nécessite un grand nombre d'images d'apprentissage (au moins plusieurs centaines, voire plusieurs milliers), ce qui soulève les mêmes inconvénients que ceux précédemment cités.

Le document : « Cheng Su et al : « Rock Classification in pétrographie thin section images based on concatenated convolutional neural networks », Arxiv.org, XP081627653, 23 mars 2020 » décrit une classification de roches sur la base d'images et d'un réseau de neurones.

### Résumé de l'invention

La présente invention a pour but de détecter des constituants géologiques d'un échantillon de roche, de manière automatique, avec un temps de préparation limité et un nombre d'opérations limité. Pour cela, la présente invention concerne un procédé de détection d'un constituant géologique d'un échantillon de roche, au moyen d'une méthode d'apprentissage automatique entraînée avec un nombre limité d'images d'apprentissage, grâce à une segmentation et à un découpage des images d'apprentissage en une pluralité de vignettes.

La présente invention concerne un procédé de détection d'au moins un constituant géologique d'un échantillon de roche, à partir d'images d'apprentissage. Pour ce procédé, on met en oeuvre les étapes suivantes :
a) On segmente lesdites images d'apprentissage en au moins deux couleurs : une première couleur pour chaque zone desdites images d'apprentissage ne comprenant aucun constituant géologique, et au moins une deuxième couleur pour chaque zone desdites mages d'apprentissage comprenant un constituant géologique ;
b) On découpe lesdites images d'apprentissage et lesdites images segmentées en une pluralité de vignettes ;
c) On entraîne un algorithme d'apprentissage automatique de classification au moyen desdites vignettes desdites images d'apprentissage et au moyen desdites vignettes desdites images d'apprentissage segmentées pour classifier lesdites vignettes desdites images d'apprentissage en fonction de ladite couleur desdites vignettes desdites images d'apprentissage segmentées correspondantes ;
d) On acquiert une image dudit échantillon de roche ;
e) On découpe ladite image acquise dudit échantillon de roche en une pluralité devignettes ; et
f) On détecte ledit au moins un constituant géologique dans chacune desdites vignettes de ladite image acquise dudit échantillon de roche en appliquant un modèle formé par ledit algorithme d'apprentissage automatique, auxdites vignettes de ladite image acquise dudit échantillon de roche.

Avantageusement, ledit constituant géologique est choisi parmi les microfossiles, les nanofossiles, les débris végétaux, les minéraux, les spores de pollen.

Selon un mode de réalisation chacune desdites au moins deuxièmes couleurs correspond à un type de constituant géologique.

Selon un aspect, le nombre d'images d'apprentissage est compris entre 3 et 20, de préférence entre 3 et 10.

Conformément à une mise en oeuvre, ledit algorithme d'apprentissage automatique utilise un réseau de neurones artificiel, de préférence un réseau de neurones convolutif ou un réseau de neurones entièrement convolutif.

De manière avantageuse, on acquiert ladite image dudit échantillon de roche à partir d'une lame mince dudit échantillon de roche.

Selon une caractéristique, on acquiert ladite image dudit échantillon de roche au moyen d'un microscope optique ou électronique, à lumière polarisée ou non, d'une photographie, d'un scanner d'une tomographie avec synchrotron, ou d'une imagerie par rayons X. Conformément un mode de réalisation, on entraîne ledit algorithme d'apprentissage automatique à classifier lesdites vignettes desdites images d'apprentissage, par analyse de la couleur du pixel central desdites vignettes desdites images segmentées. Avantageusement, on découpe ladite image acquise en une pluralité de vignettes en mettant en oeuvre, pour chaque pixel de ladite image acquise, un découpage d'une vignette entourant ledit pixel, et on utilise ledit apprentissage automatique entraîné pour déterminer si ledit pixel appartient audit constituant géologique.

Selon une mise en oeuvre, on entraîne ledit algorithme d'apprentissage automatique pour segmenter toute la surface desdites vignettes desdites images d'apprentissage pour y délimiter lesdits constituants géologiques.

De manière avantageuse, on détecte lesdits constituants géologiques en délimitant pour chaque vignette de ladite image acquise lesdits constituants géologiques.

Selon une mise en oeuvre, ledit procédé de détection comporte au moins une étape supplémentaire choisie parmi :
- On compte le nombre de constituants géologiques de ladite image acquise dudit échantillon de roche à partir de la détection dudit constituant géologique, ou
- On détermine la proportion du volume de l'échantillon de roche occupée par les constituants géologique à partir de la détection dudit constituant géologique, ou
- On estime des caractéristiques morphologiques et/ou texturales desdites constituants géologiques, ou
- On applique une méthode de classification supervisée pour catégoriser lesdits constituants géologiques depuis la détection dudit constituant géologique.

De plus, l'invention concerne un procédé d'exploitation d'un sol ou d'un sous-sol, dans lequel on met en oeuvre les étapes suivantes :
a) On détecte au moins un constituant géologique d'un échantillon de roche au moyen du procédé de détection selon l'une des caractéristiques précédentes ; et
b) On exploite ledit sol ou ledit sous-sol en fonction de ladite détection dudit constituant géologique de ladite au moins une roche.

Selon un mode de réalisation, ladite exploitation du sol ou du sous-sol concerne la construction d'un ouvrage sur ledit sol ou dans le sous-sol, le stockage de gaz dans le sous-sol, ou l'exploitation de matières premières dudit sol ou dudit sous-sol, de préférence lesdites matières premières étant la roche elle-même, ou un matériau ou un fluide contenu dans ledit sol ou dans ledit sous-sol.

En outre, l'invention concerne un procédé de détermination du climat dans une zone géographique à travers les âges géologiques, dans lequel on met en oeuvre les étapes suivantes :
a) On prélève au moins deux échantillons de roche à différentes profondeurs d'une formation souterraine ;
b) On détecte au moins un constituant géologique pour chaque échantillon de roche au moyen du procédé de détection selon l'une des caractéristiques précédentes ; et
c) On détermine ledit climat ainsi que l'âge géologique dans ladite zone géographique en fonction dudit au moins un constituant géologique détecté.

D'autres caractéristiques et avantages du procédé selon l'invention, apparaîtront à la lecture de la description ci-après d'exemples non limitatifs de réalisations, en se référant aux figures annexées et décrites ci-après.

### Liste des figures

La figure 1 illustre les étapes du procédé de détection selon un mode de réalisation de l'invention.
La figure 2 illustre une première variante de la méthode d'apprentissage automatique utilisée par le procédé de détection selon l'invention.
La figure 3 illustre une deuxième variante de la méthode d'apprentissage automatique utilisée par le procédé selon l'invention.
La figure 4 illustre une image acquise d'un échantillon de roche ainsi que les zones dans lesquelles des constituants géologiques ont été détectés au moyen du procédé selon un mode de réalisation de l'invention.

### Description des modes de réalisation

La présente invention concerne un procédé de détection de constituants géologiques d'un échantillon de roche. On appelle échantillon de roche un morceau de roche, par exemple (non limitatif) prélevé par carottage. On entend par constituants géologiques au moins un constituant inclus au sein de la roche. Un tel constituant géologique de roches peut être choisi parmi les microfossiles, les nanofossiles, les débris végétaux, les minéraux, les spores de pollen, ou tout élément analogue. On appelle détection de constituants géologiques le repérage des constituants géologiques au sein de l'échantillon de roche

Le procédé de détection selon l'invention se base sur l'utilisation d'une image de l'échantillon de roche et sur la mise en oeuvre d'un algorithme d'apprentissage automatique (de l'anglais « machine learning ») pour automatiser cette détection.

Selon un mode de réalisation de l'invention, l'image acquise de l'échantillon de roche, et l'ensemble des images d'apprentissage mises en oeuvre pour l'entraînement de l'algorithme d'apprentissage peuvent être de tous types.

Conformément à une mise en oeuvre de l'invention, l'image peut être celle d'un échantillon de roche entier. Alternativement, l'image peut être celle d'une lame mince de l'échantillon de roche. Les échantillons observés peuvent notamment être des lames dites « lames minces » de trois types : lames de matériaux de très faibles épaisseurs de l'ordre de 30 µm, lames plus épaisses de l'ordre de 100 µm, ou sections polies dont l'épaisseur peut aller jusqu'à 10 mm. Pour pouvoir observer correctement ces différents types d'échantillons au microscope, il est important de disposer d'une surface plane et d'une très faible rugosité (quasiment lisse).

De plus, l'image peut être obtenue de différente manière, par exemple au moyen d'un microscope à lumière polarisée ou non, au moyen d'un scanner, au moyen d'une photographie, ou tout système analogue.

Selon un mode de réalisation préféré de l'invention, l'image peut être obtenue par un microscope à partir d'une lame mince de l'échantillon de roche.

Selon l'invention, le procédé de détection comprend les étapes suivantes :
1- Entrainement de l'algorithme d'apprentissage automatique
2 - Acquisition de l'image de l'échantillon de roche
3 - Détection des constituants géologiques de l'échantillon de roche

De manière un peu plus détaillée, le procédé selon l'invention comprend les étapes suivantes :
1- Entrainement de l'algorithme d'apprentissage automatique
   1.1- Segmentation des images d'apprentissage
   1.2- Découpage des images d'apprentissage et des images segmentées
   1.3- Classification
2- Acquisition de l'image de l'échantillon de roche
3 - Détection des constituants géologiques de l'échantillon de roche
   3.1 - Découpage de l'image acquise
   3.2 - Détection des constituants

L'étape 1 et les sous-étapes associées 1.1 à 1.3 peuvent réalisées hors ligne, une seule fois et au préalable. Les étapes 2 et 3, et les sous étapes associées 3.1 et 3.2, peuvent être réalisées en ligne, pour chaque échantillon de roche considéré. En d'autres termes, si l'on souhaite détecter les constituants géologiques de plusieurs échantillons de roches, on répète les étapes 2 et 3 pour chaque échantillon de roche, l'algorithme d'apprentissage automatique n'ayant besoin d'être entraîné qu'une seule fois. Les étapes seront détaillées dans la suite de la description.

Le procédé selon l'invention peut être mis en oeuvre au moyen d'un système informatique, notamment un ordinateur, en particulier les étapes 1.3, 2, et 3 (3.1 et 3.2).

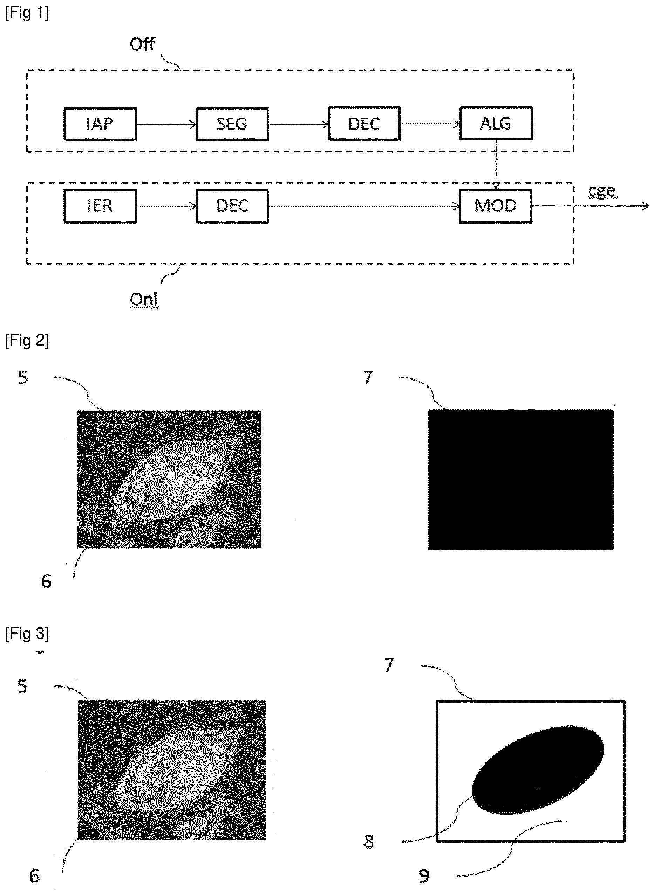

La figure 1 illustre, schématiquement et de manière non limitative, les étapes du procédé de détection selon un mode de réalisation de l'invention. Dans un premier temps, on réalise les étapes hors ligne Off à partir des images d'apprentissage IAP. On segmente SEG chaque image d'apprentissage IAP en au moins deux couleurs : une couleur étant associée aux zones sans constituants géologiques, et au moins une autre couleur étant associée aux zones avec constituants géologiques. Ensuite, on découpe DEC chaque image d'apprentissage IAP et chaque image segmentée en une pluralité de vignettes. On entraîne ensuite l'algorithme d'apprentissage automatique ALG par classification des vignettes. En ligne Onl, on acquiert une image de l'échantillon de roche IER. On découpe DEC ensuite l'image d'échantillon de roche IER en une pluralité de vignettes. En appliquant le modèle MOD résultant de l'algorithme d'apprentissage automatique ALG aux vignettes de l'image acquise d'échantillon de roche IER, on détecte les constituants géologiques cge de l'échantillon de roche.

### 1- Entraînement de l'algorithme d'apprentissage automatique

Il s'agit lors de cette étape d'entraîner l'algorithme d'apprentissage automatique afin de pouvoir automatiser la détection des constituants géologiques à partir des images d'apprentissage. A la fin de cette étape, l'algorithme d'apprentissage automatique forme un modèle, qui a pour entrée une image d'échantillon de roche et en sortie la détection des constituants géologiques.

Le procédé selon l'invention nécessite peu d'images d'apprentissage en raison de la segmentation et du découpage des images d'apprentissage en vignettes, étapes qui seront décrites ci-après. Ainsi, on réduit le temps et les opérations nécessaires à l'entraînement de l'algorithme. Avantageusement, le nombre d'images d'apprentissage peut être compris entre 3 et 20, de préférence entre 3 et 10 et peut valoir 5. Par exemple, il est évident que l'acquisition de 5 images de lames minces au microscope est 60 fois plus rapide que l'acquisition de 300 images de lame mince au microscope, qui sont utiles pour les procédés selon l'art antérieur utilisant plusieurs centaines d'images d'apprentissage. Si on évalue à une dizaine de minutes chaque acquisition d'une image de lame mince au microscope, le gain dans ce cas est de 2950 min soit plus de 49h. Ainsi, le procédé selon l'invention est moins fastidieux et plus rapide.

De plus, le procédé selon l'invention peut comprendre une étape préalable d'acquisition des images d'apprentissage, cette étape pouvant être mise en oeuvre de la même manière que l'étape 2 d'acquisition de l'image de l'échantillon de roche. Par exemple, cette étape préalable peut consister en la préparation de lames minces et en la réalisation de microscopie de ces lames minces pour former les images d'apprentissage.

Selon un mode de réalisation de l'invention, l'algorithme d'apprentissage automatique peut être un algorithme d'apprentissage automatique de classification supervisée, par exemple un réseau de neurones artificiel, de préférence un réseau de neurones convolutifs (CNN de l'anglais « Convolutive Neural Network ») ou un réseau de neurones entièrement convolutifs (FCNN de l'anglais « Fully Convolutive Neural Network »). Le réseau de neurones convolutifs et le réseau de neurones entièrement convolutifs sont particulièrement adaptés au traitement de l'image.

Alternativement, l'algorithme d'apprentissage automatique peut être de tout type, notamment un réseau de neurones non convolutifs, une méthode de forêt aléatoire, une méthode de machine à vecteurs de support (de l'anglais « Support Vecteur Machine » ou SVM), une méthode de processus gaussiens.

### 1.1 - Segmentation des images d'apprentissage

Lors de cette étape, on segmente lesdites images d'apprentissage en au moins deux couleurs :
- Une première couleur pour les zones des images d'apprentissage sans constituant géologique,
- Au moins une deuxième couleur pour les zones des images d'apprentissage avec constituant géologique.

Selon un mode de réalisation de l'invention, on peut segmenter les images d'apprentissage en uniquement deux couleurs : tous les constituants géologiques ont alors la même couleur. Le procédé de détection permet alors de détecter tous les constituants géologiques. Pour ce mode de réalisation, la première couleur peut être le noir et la deuxième couleur peut être le blanc (ou inversement); ainsi le contraste est amélioré,.

Alternativement, on peut segmenter les images d'apprentissage en un nombre de couleurs supérieur à deux. Dans ce cas, on peut attribuer une couleur par type de constituants géologiques. Selon un premier exemple, on peut attribuer une couleur pour les microfossiles, et une couleur pour les débris végétaux, etc. Selon un deuxième exemple, on peut attribuer une couleur par différents types de microfossiles, ou par différents types de débris végétaux, etc. En outre, il est possible de combiner ces exemples : on peut attribuer une couleur par différents types de microfossiles, et une couleur par différents types de débris végétaux, etc.

### 1.2 - Découpage des images d'apprentissage et des images segmentées

Lors de cette étape, on découpe les images d'apprentissage et les images segmentées obtenues à l'étape précédente en une pluralité de vignettes. On appelle vignette (ou en anglais « patch ») une partie de l'image d'apprentissage ou de l'image segmentée, ainsi les vignettes sont formées d'un ensemble de pixels adjacents de l'image d'apprentissage ou de l'image segmentée. On obtient alors deux jeux de vignettes : un premier jeu de vignettes des images d'apprentissage et un deuxième jeu de vignettes des images segmentées. Ainsi, le procédé selon l'invention n'entraine pas directement l'algorithme d'apprentissage automatique à repérer les constituants géologiques sur des images entières, mais simplement à travailler sur de petits morceaux d'image (vignettes). D'une certaine manière, on remplace une tâche complexe pour laquelle on a peu de données d'entrainement, en une série de tâches plus simples et pour lesquelles on peut générer davantage de données, ce qui permet de diminuer le nombre d'images d'apprentissage.

Selon un mode de réalisation de l'invention, les vignettes peuvent avoir sensiblement une forme rectangulaire, voire carré. Toutefois, les vignettes peuvent avoir toutes autres formes.

De préférence, la taille des vignettes peut correspondre sensiblement aux dimensions des constituants géologiques. Par exemple, la taille minimale des vignettes peut correspondre sensiblement à la taille d'un rectangle qui englobe le plus grand constituant géologique.

Selon un aspect de l'invention, toutes les vignettes peuvent comporter le même nombre de pixels pour faciliter l'apprentissage automatique. Ainsi, il n'est pas nécessaire de prévoir un redimensionnement de la taille des vignettes pour l'apprentissage automatique. En variante, les vignettes ont des nombres différents de pixels.

De manière à améliorer l'apprentissage de l'algorithme d'apprentissage automatique et par conséquent la précision du procédé de détection selon l'invention, on peut découper les images d'apprentissage, de manière à former, pour la totalité des images d'apprentissage, au moins cent vignettes, de préférence au moins plusieurs centaines de vignettes.

Lors de cette étape, on peut, de préférence, découper les images d'apprentissage initiales et les images d'apprentissage segmentées de manière identique. Ainsi, la qualité de l'apprentissage est améliorée. En effet, on souhaite entraîner l'apprentissage automatique à reproduire la correspondance entre l'image d'apprentissage et l'image segmentée.

### 1.3 - Classification

Lors de cette étape, on entraîne l'algorithme d'apprentissage automatique de classification supervisée au moyen des vignettes découpées des images d'apprentissage et des images segmentées pour classifier les vignettes des images d'apprentissage, en fonction de la couleur (ou le cas échéant des couleurs) des vignettes des images segmentées correspondantes. En d'autres termes, chaque zone des images d'apprentissage, pour laquelle la vignette de l'image segmentée correspondante est de la première couleur est définie comme une zone sans constituant géologique, et chaque zone des images d'apprentissage, pour laquelle la vignette de l'image segmentée correspondante est, au moins partiellement, de l'au moins deuxième couleur, est définie comme une zone avec constituant géologique.

L'entraînement de l'algorithme d'apprentissage automatique peut être mis en oeuvre notamment par une des méthodes suivantes :
- La méthode décrite dans le document Ciresan, D., Giusti, A., Gambardella, L. M., & Schmidhuber, J. [2012]. Deep neural networks segment neuronal membranes in electron microscopy images. In Advances in neural information processing systems, 2843-2851. Pour cette méthode, on apprend à un réseau de neurones, si possible convolutif (CNN de l'anglais « Convolutive Neural Network »), à classifier des vignettes en recherchant si leur pixel central appartient à un constituant recherché ou non. Puis, cette capacité est appliquée pixel par pixel sur l'image acquise pour délimiter les constituants recherchés. Dans la suite de la description, on appelle cette méthode, la méthode « basée pixels ».
- La méthode décrite dans le document Ronneberger O., Fischer P., Brox T. [2015] U-Net: Convolutional Networks for Biomedical Image Segmentation. In: Navab N., Hornegger J., Wells W., Frangi A. (eds) Medical Image Computing and Computer-Assisted Intervention - MICCAI 2015. MICCAI 2015. Lecture Notes in Computer Science, vol 9351. Springer, Cham. Pour cette méthode, on apprend à un réseau de neurones, si possible entièrement convolutif (FCNN de l'anglais « Fully Convolutive Neural Network ») à segmenter toute la surface des vignettes pour y délimiter les constituants recherchés. Puis, cette capacité est appliquée sur de nouvelles images en les découpant en des vignettes de taille similaire et en travaillant vignette par vignette. Dans la suite de la description, on appelle cette méthode, la méthode « basée vignettes ».

Ainsi, pour le mode de réalisation, pour lequel on utilise la méthode « basée pixels », on peut mettre en oeuvre cette étape par analyse de la couleur du pixel central des vignettes des images segmentées. De cette manière, on entraine un réseau de neurones artificiel à classifier automatiquement les vignettes non-segmentées (des images d'apprentissage) selon au moins deux catégories : soit le pixel central est d'au moins de la deuxième couleur (c'est-à-dire n'est pas de la première couleur) sur l'image segmentée (c'est-à-dire appartient à un des constituants géologiques qu'on souhaite repérer), soit le pixel central est de la première couleur sur l'image segmentée (c'est-à-dire n'appartient pas à un des constituants géologiques qu'on souhaite repérer). De préférence, on peut utiliser une méthode d'apprentissage automatique profond avec un réseau de neurones convolutif (CNN). D'une manière générale, la méthode d'apprentissage automatique peut alternativement être choisie parmi la gamme des méthodes de classification supervisée fonctionnant sur les images. La figure 2 illustre, schématiquement et de manière non limitative, deux vignettes d'une même zone. La vignette 5 de la figure de gauche est la vignette de l'image d'apprentissage. Cette vignette 5 comprend un constituant géologique 6. La vignette 7 de la figure de droite est la vignette de l'image segmentée, cette vignette 7 est de la deuxième couleur (ici noire) en raison de la présence du constituant géologique 6 dans la zone correspondante.

Ainsi, pour le mode de réalisation, pour lequel on applique la méthode « basée vignettes », on peut mettre en oeuvre cette étape par segmentation de la totalité de la surface des vignettes pour y délimiter les constituants géologiques. De cette manière, on entraine un apprentissage automatique à segmenter automatiquement les vignettes non-segmentées des images d'apprentissage selon les catégories déjà définies, c'est-à-dire au moins deux : première couleur (c'est-à-dire n'appartient pas à un des constituants géologiques qu'on souhaite repérer) ou au moins deuxième couleur (c'est-à-dire appartient à un des constituants géologiques qu'on souhaite repérer). De préférence, on peut utiliser une méthode d'apprentissage profond avec un réseau de neurones entièrement convolutif (FCNN). La figure 3 illustre, schématiquement et de manière non limitative deux vignettes d'une même zone. La vignette 5 de la figure de gauche est la vignette de l'image d'apprentissage. Cette vignette 5 comprend un constituant géologique 6. La vignette 7 de la figure de droite est la vignette de l'image segmentée, cette vignette 7 comprend une zone 8 de la deuxième couleur (noire) qui correspond au constituant géologique 6 de la vignette d'apprentissage 5. Cette vignette 7 comprend une zone 9 de la première couleur (blanc) qui correspond à une zone sans constituant géologique de l'image d'apprentissage.

### 2 - Acquisition de l'image de l'échantillon de roche

Il s'agit lors de cette étape d'acquérir une image de l'échantillon de roche à analyser. Pour cela, on peut mettre en oeuvre, une des méthodes d'acquisition d'image adaptée au procédé selon l'invention : microscope optique ou électronique, photographie, scanner, tomographie avec synchrotron, imagerie par rayons X, etc. De plus, on peut acquérir une image directement de l'échantillon de roche ou à partir d'une lame mince de l'échantillon de roche.

Selon un mode de réalisation de l'invention, lorsque le procédé de détection selon l'invention met en oeuvre des images de lame mince, cette étape peut comporter une sous-étape de préparation d'une lame-mince à partir de l'échantillon de roche à analyser.

Selon un aspect de l'invention, on acquiert une image de l'échantillon de roche plus grande que la zone à étudier de l'échantillon de roche, afin d'éviter les effets de bord du procédé de détection.

### 3 - Détection des constituants géologiques de l'échantillon de roche

Lors de cette étape, on détecte les constituants géologiques de l'échantillon de roche, au moyen de l'image acquise de l'échantillon de roche, et au moyen du modèle formé par l'algorithme d'apprentissage automatique. Ainsi, on applique le modèle formé par l'algorithme d'apprentissage automatique à l'image acquise de l'échantillon de roche.

### 3.1 - Découpage de l'image acquise

Lors de cette étape, on découpe l'image acquise de l'échantillon de roche en une pluralité de vignettes afin d'appliquer le modèle formé par l'algorithme d'apprentissage automatique obtenu à l'étape 1 à chaque vignette de l'image acquise de l'échantillon de roche (obtenue à l'étape 2).

Pour le mode de réalisation pour lequel on utilise la méthode « basée pixels », on peut mettre en oeuvre cette étape, au moyen d'un découpage de l'image acquise de l'échantillon de roche en une pluralité de vignettes en mettant en oeuvre, pour chaque pixel de l'image acquise, un découpage d'une vignette entourant le pixel. Selon une option de ce mode de réalisation, les vignettes de l'image acquise peuvent avoir sensiblement la même taille que les vignettes des images d'apprentissage et des images segmentées utilisées pour entrainer l'algorithme d'apprentissage automatique (à l'étape 1). Cette option de réalisation permet de donner des meilleurs résultats.

Pour le mode de réalisation pour lequel on utilise la méthode « basée vignettes », on peut mettre en oeuvre cette étape, au moyen d'un découpage de l'image acquise en vignettes qui couvrent la totalité de l'image acquise. Selon une option de ce mode de réalisation, les vignettes de l'image acquise peuvent avoir sensiblement la même taille que les vignettes des images d'apprentissage et des images segmentées utilisées pour entraîner l'algorithme d'apprentissage automatique (à l'étape 1). Cette option de réalisation permet de donner des meilleurs résultats.

### 3.2 - Détection des constituants géologiques

Lors de cette étape, on détecte les constituants géologiques de l'échantillon de roche au moyen du modèle obtenu par l'algorithme d'apprentissage automatique (étape 1) appliqué aux vignettes de l'image acquise (étape 3.1). A l'issue de cette étape, on obtient la segmentation complète de l'image acquise, avec les couleurs utilisées à l'étape 1.1. On détecte alors les constituants géologiques de l'image acquise aux pixels, qui ont pour couleur l'au moins deuxième couleur (en d'autres termes qui n'ont pas la première couleur).

Pour le mode de réalisation, pour lequel on utilise la méthode « basée pixels », on peut mettre en oeuvre cette étape, en identifiant pour chaque vignette de l'image acquise si le pixel central fait partie d'un constituant géologique ou non, au moyen du modèle formé par l'algorithme d'apprentissage automatique. On peut alors colorer ce pixel soit de la première couleur (si ce pixel ne fait pas partie d'un constituant géologique) soit d'une autre couleur, par exemple la deuxième couleur (si ce pixel fait partie d'un constituant géologique). A l'issue de cette étape, après avoir appliqué cette méthode à chaque pixel de l'image acquise, on obtient la segmentation en couleurs de l'image acquise, avec les zones de l'au moins deuxième couleur correspondant aux constituants géologiques que l'on détecte.

Pour le mode de réalisation pour lequel on utilise la méthode « basée vignettes », on peut mettre en oeuvre cette étape, en délimitant pour chaque vignette de ladite image acquise lesdits constituants géologiques au moyen du modèle formé par l'algorithme d'apprentissage automatique. En d'autres termes pour chaque vignette de l'image acquise, on délimite, le cas échéant, un constituant géologique au sein de la vignette, et on attribue au moins une deuxième couleur à l'éventuel constituant géologique. A l'issue de cette étape, après avoir appliqué cette méthode à chaque vignette de l'image acquise, on obtient la segmentation en couleurs de l'image acquise, avec des zones de l'au moins deuxième couleur correspondant aux constituants géologiques que l'on détecte.

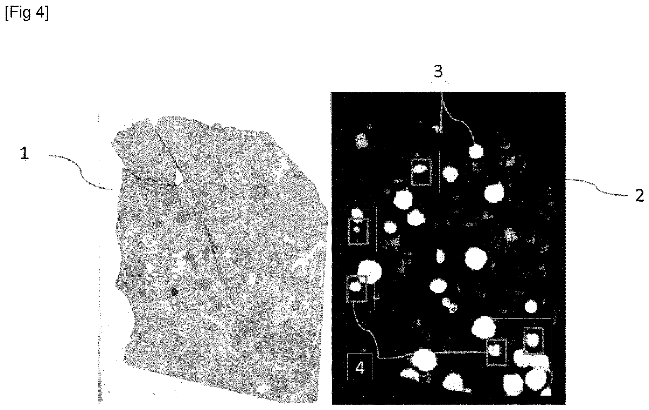

La figure 4 illustre, schématiquement et de manière non limitative, dans sa partie gauche, une image 1 acquise d'un échantillon de roche, et dans sa partie droite l'image segmentée 2 de l'image 1. Cette image acquise 1 a été obtenue par microscopie d'une lame mince de l'échantillon de roche. L'image segmentée 2 a été obtenue par le procédé de détection selon l'invention. Pour le procédé de détection selon l'invention, l'algorithme d'apprentissage automatique a été entraîné avec cinq images d'apprentissage. De plus, on a mis en oeuvre un réseau de neurones convolutifs et la méthode « basée pixels ». Sur l'image segmentée 2, le noir correspond à la première couleur, c'est-à-dire les zones sans constituants géologiques, et le blanc correspond à la deuxième couleur, c'est-à-dire les zones avec constituants géologiques 3. Sur cette figure, on a également illustré par les rectangles 4 les constituants géologiques qui n'avaient pas été identifiés par un opérateur lors de l'étude de cette lame mince. Ainsi, on remarque que le procédé selon l'invention peut être plus précis qu'un opérateur, en identifiant automatiquement tous les constituants géologiques.

De plus, le procédé selon l'invention peut comporter au moins une des étapes supplémentaires suivantes :
- On compte le nombre de constituants géologiques de l'image acquise de l'échantillon de roche à partir de la détection dudit constituant géologique, en d'autres termes à partir de l'image acquise segmentée, et/ou
- On détermine la proportion du volume de l'échantillon de roche occupée par les constituants géologique à partir de la détection dudit constituant géologique, en d'autres termes à partir de l'image acquise segmentée, et/ou
- On estime des caractéristiques morphologiques et/ou texturales (par exemple la forme, les dimensions, la présence de stries, etc.) des constituants géologiques, par exemple au moyen d'un traitement d'image, et/ou
- On applique une méthode de classification supervisée pour catégoriser les constituants géologiques depuis la détection du constituant géologique. Par exemple, on peut déterminer l'espèce à laquelle appartient chaque microfossile détecté. Cette étape peut nécessiter d'avoir préalablement entrainé un réseau de neurones artificiel à effectuer cette catégorisation, à partir d'une base d'images dédiée, et de manière indépendante de l'algorithme d'apprentissage du procédé de détection selon l'invention.

Ces étapes peuvent être réalisées automatiquement, de préférence grâce à des moyens informatiques, tels qu'un ordinateur.

En outre, l'invention concerne un procédé d'exploitation du sol ou du sous-sol. Pour ce procédé, on met en oeuvre les étapes suivantes :
a) On détecte au moins un constituant géologique d'un échantillon de roche au moyen du procédé de détection selon l'une quelconque des variantes ou des combinaisons de variantes décrites précédemment ; et
b) On exploite le sol et/ou le sous-sol en fonction des constituants géologiques de l'échantillon de roche détectés à l'étape précédente.

Pour ce procédé d'exploitation du sol ou du sous-sol, le procédé peut comprendre une étape préalable de prélèvement d'un échantillon de roche du sol ou du sous-sol.

L'exploitation peut concerner notamment le domaine de la construction de bâtiments ou d'ouvrages d'art, ou le domaine de l'exploitation de matières premières, ou le domaine du stockage de gaz, le domaine de la détermination des risques, etc.

Dans le domaine de la construction, on détermine la constitution des affleurements rocheux et/ou du sous-sol par la catégorisation de la roche, et on réalise la construction en adaptant notamment les fondations, la structure de la construction en fonction de la catégorisation de la roche. Pour ces applications, la roche à catégoriser peut être prélevée sur le sol ou dans le sous-sol de manière peu enfouie.

Dans le domaine d'exploitation de matières premières (par exemple pour les carrières, les mines, la récupération des hydrocarbures, etc.), on détermine la constitution des affleurements rocheux et/ou du sous-sol par la détection des constituants géologique de la roche, et on réalise l'exploitation de matières premières (les matières premières peuvent être la roche elle-même, un matériau, par exemple un métal, ou un fluide, par exemple des hydrocarbures, présents dans le sous-sol), en permettant notamment de déterminer les zones adéquates (c'est-à-dire les zones de forage, les zones à creuser pour les mines ou les carrières, etc. dans le but de récupérer des matières premières), de déterminer les méthodes et les outils à utiliser (par exemple la récupération assistée des hydrocarbures, les outils de forage, la nature des engins explosifs pour les mines ou les carrières, etc.). Pour ces applications, la roche à catégoriser peut être prélevée en profondeur dans le sous-sol, peut résulter d'un débris de forage, ou peut provenir d'un effleurement, etc.

Dans le domaine du stockage de gaz, par exemple du CO₂, on détermine la constitution du sous-sol par la catégorisation de la roche, et on réalise le stockage de gaz dans le sous-sol dans une zone adéquate, c'est-à-dire dans une zone souterraine apte à stocker le gaz sans fuite.

Dans le domaine de la détermination des risques, on détermine la constitution d'un affleurement rocheux (falaise) par la catégorisation de la roche, et on réalise une opération de consolidation s'il existe un risque d'affaissement ou d'éboulement de l'affleurement rocheux.

Ainsi, ce procédé permet de mettre en oeuvre l'exploitation du sol et/ou du sous-sol, de manière simple et rapide, sans faire appel à un géologue expert. Le procédé permet également de traiter plus rapidement de très importantes quantités de roches.

L'invention concerne également un procédé de détermination du climat dans une zone géographique à travers les âges géologiques, dans lequel on met en oeuvre les étapes suivantes :
a) On prélève au moins deux échantillons de roche à différentes profondeurs d'une formation souterraine, les échantillons de roche pouvant provenir d'une même succession de dépôts rocheux ;
b) On détecte au moins un constituant géologique pour chaque échantillon de roche au moyen du procédé de détection selon l'une quelconque des variantes ou des combinaisons de variantes décrites précédemment ; et
c) On détermine ledit climat ainsi que l'âge géologique dans la zone géographique en fonction dudit au moins un constituant géologique détecté.

Comme il va de soi, l'invention ne se limite pas aux seules formes de réalisation décrites ci-dessus à titre d'exemple, elle embrasse au contraire toutes les variantes de réalisation.

## Revendications

1. Procédé de détection d'au moins un constituant géologique (cge) d'un échantillon de roche, à partir d'images d'apprentissage, **caractérisé en ce qu'**on met en oeuvre les étapes suivantes au moyen d'un système informatique :
a) On segmente (SEG) lesdites images d'apprentissage (IAP) en au moins deux couleurs : une première couleur pour chaque zone desdites images d'apprentissage (IAP) ne comprenant aucun constituant géologique, et au moins une deuxième couleur pour chaque zone desdites mages d'apprentissage (IAP) comprenant un constituant géologique (3) ;
b) On découpe (DEC) lesdites images d'apprentissage (IAP) et lesdites images segmentées en une pluralité de vignettes ;
c) On entraîne un algorithme d'apprentissage automatique (ALG) de classification au moyen desdites vignettes desdites images d'apprentissage (IAP) et au moyen desdites vignettes desdites images d'apprentissage segmentées pour classifier lesdites vignettes desdites images d'apprentissage (IAP) en fonction de ladite couleur desdites vignettes desdites images d'apprentissage segmentées correspondantes ;
d) On acquiert une image dudit échantillon de roche (IER) ;
e) On découpe (DEC) ladite image acquise dudit échantillon de roche (IER) en une pluralité de vignettes ; et
f) On détecte ledit au moins un constituant géologique (cge) dans chacune desdites vignettes de ladite image acquise dudit échantillon de roche (IER) en appliquant un modèle (MED) formé par ledit algorithme d'apprentissage automatique (ALG), auxdites vignettes de ladite image acquise dudit échantillon de roche (IER).

2. Procédé de détection selon la revendication 1, dans lequel ledit constituant géologique (cge) est choisi parmi les microfossiles, les nanofossiles, les débris végétaux, les minéraux, les spores de pollen.

3. Procédé de détection selon l'une des revendications précédentes, dans lequel chacune desdites au moins deuxièmes couleurs correspond à un type de constituant géologique (cge).

4. Procédé de détection selon l'une des revendications précédentes, dans lequel le nombre d'images d'apprentissage (IAP) est compris entre 3 et 20, de préférence entre 3 et 10.

5. Procédé de détection selon l'une des revendications précédentes, dans lequel ledit algorithme d'apprentissage automatique (ALG) utilise un réseau de neurones artificiel, de préférence un réseau de neurones convolutif ou un réseau de neurones entièrement convolutif.

6. Procédé de détection selon l'une des revendications précédentes, dans lequel on acquiert ladite image dudit échantillon de roche (IER) à partir d'une lame mince dudit échantillon de roche.

7. Procédé de détection selon l'une des revendications précédentes, dans lequel on acquiert ladite image dudit échantillon de roche (IER) au moyen d'un microscope optique ou électronique, à lumière polarisée ou non, d'une photographie, d'un scanner d'une tomographie avec synchrotron, ou d'une imagerie par rayons X.

8. Procédé de détection selon l'une des revendications précédentes, dans lequel on entraîne ledit algorithme d'apprentissage automatique (ALG) à classifier lesdites vignettes desdites images d'apprentissage (IAP), par analyse de la couleur du pixel central desdites vignettes desdites images segmentées.

9. Procédé de détection selon la revendication 8, dans lequel on découpe ladite image acquise dudit échantillon de roche (IER) en une pluralité de vignettes en mettant en oeuvre, pour chaque pixel de ladite image acquise dudit échantillon de roche (IER), un découpage d'une vignette entourant ledit pixel, et on utilise ledit modèle (MOD) formé par ledit apprentissage automatique (ALG) pour déterminer si ledit pixel appartient audit constituant géologique (cge).

10. Procédé de détection selon l'une des revendications 1 à 7, dans lequel on entraîne ledit algorithme d'apprentissage automatique (ALG) pour segmenter toute la surface desdites vignettes desdites images d'apprentissage pour y délimiter lesdits constituants géologiques.

11. Procédé de détection selon la revendication 10, dans lequel on détecte lesdits constituants géologiques (cge) en délimitant pour chaque vignette de ladite image acquise dudit échantillon de roche (IER) lesdits constituants géologiques.

12. Procédé de détection selon l'une des revendications précédentes, dans lequel ledit procédé de détection comporte au moins une étape supplémentaire choisie parmi :
- On compte le nombre de constituants géologiques (cge) de ladite image acquise dudit échantillon de roche (IER) à partir de la détection dudit constituant géologique, ou
- On détermine la proportion du volume de l'échantillon de roche occupée par les constituants géologique (cge) à partir de la détection dudit constituant géologique, ou
- On estime des caractéristiques morphologiques et/ou texturales desdites constituants géologiques (cge), ou
- On applique une méthode de classification supervisée pour catégoriser lesdits constituants géologiques (cge) depuis la détection dudit constituant géologique.

13. Procédé d'exploitation d'un sol ou d'un sous-sol, dans lequel on met en oeuvre les étapes suivantes :
a) On détecte au moins un constituant géologique (cge) d'un échantillon de roche au moyen du procédé de détection selon l'une des revendications précédentes ; et
b) On exploite ledit sol ou ledit sous-sol en fonction de ladite détection dudit constituant géologique (cge) de ladite au moins une roche.

14. Procédé d'exploitation d'un sol ou d'un sous-sol selon la revendication 13, dans lequel ladite exploitation du sol ou du sous-sol concerne la construction d'un ouvrage sur ledit sol ou dans le sous-sol, le stockage de gaz dans le sous-sol, ou l'exploitation de matières premières dudit sol ou dudit sous-sol, de préférence lesdites matières premières étant la roche elle-même, ou un matériau ou un fluide contenu dans ledit sol ou dans ledit sous-sol.

15. Procédé de détermination du climat dans une zone géographique à travers les âges géologiques, dans lequel on met en oeuvre les étapes suivantes :
a) On prélève au moins deux échantillons de roche à différentes profondeurs d'une formation souterraine ;
b) On détecte au moins un constituant géologique (cge) pour chaque échantillon de roche au moyen du procédé de détection selon l'une des revendications 1 à 12 ; et
c) On détermine ledit climat ainsi que l'âge géologique dans ladite zone géographique en fonction dudit au moins un constituant géologique (cge) détecté.

## Patentansprüche

1. Verfahren zur Erkennung mindestens eines geologischen Bestandteils (cge) einer Gesteinsprobe ausgehend von Lernbildern, **dadurch gekennzeichnet, dass** die folgenden Schritte mittels eines EDV-Systems durchgeführt werden:
a) die Lernbilder (IAP) werden in mindestens zwei Farben segmentiert (SEG): eine erste Farbe für jede Zone der Lernbilder (IAP), die keinen geologischen Bestandteil enthält, und mindestens eine zweite Farbe für jede Zone der Lernbilder (IAP), die einen geologischen Bestandteil (3) enthält;
b) die Lernbilder (IAP) und die segmentierten Bilder werden in eine Vielzahl von Miniaturbildern zerlegt (DEC);
c) ein automatischer Lernalgorithmus (ALG) der Klassifizierung mittels der Miniaturbilder der Lernbilder (IAP) und mittels der Miniaturbilder der segmentierten Lernbilder wird trainiert, um die Miniaturbilder der Lernbilder (IAP) abhängig von der Farbe der Miniaturbilder der entsprechenden segmentierten Lernbilder zu klassifizieren;
d) ein Bild der Gesteinsprobe (IER) wird erfasst;
e) das erfasste Bild der Gesteinsprobe (IER) wird in eine Vielzahl von Miniaturbildern zerlegt (DEC); und
f) der mindestens eine geologische Bestandteil (cge) wird in jedem der Miniaturbilder des erfassten Bilds der Gesteinsprobe (IER) erkannt, indem ein vom automatischen Lernalgorithmus (ALG) gebildetes Modell (MED) an die Miniaturbilder des erfassten Bilds der Gesteinsprobe (IER) angewendet wird.

2. Erkennungsverfahren nach Anspruch 1, wobei der geologische Bestandteil (cge) unter den Mikrofossilen, den Nanofossilen, den Pflanzenresten, den Mineralien, den Sporen von Pollen ausgewählt wird.

3. Erkennungsverfahren nach einem der vorhergehenden Ansprüche, wobei jede der mindestens zweiten Farben einem Typ von geologischem Bestandteil (cge) entspricht.

4. Erkennungsverfahren nach einem der vorhergehenden Ansprüche, wobei die Anzahl von Lernbildern (IAP) zwischen 3 und 20, vorzugsweise zwischen 3 und 10 liegt.

5. Erkennungsverfahren nach einem der vorhergehenden Ansprüche, wobei der automatische Lernalgorithmus (ALG) ein künstliches neuronales Netzwerk verwendet, vorzugsweise ein gefaltetes neuronales Netzwerk oder ein voll gefaltetes neuronales Netzwerk.

6. Erkennungsverfahren nach einem der vorhergehenden Ansprüche, wobei das Bild der Gesteinsprobe (IER) ausgehend von einem Dünnschliff der Gesteinsprobe erfasst wird.

7. Erkennungsverfahren nach einem der vorhergehenden Ansprüche, wobei das Bild der Gesteinsprobe (IER) mittels eines optischen oder elektronischen Mikroskops, mit polarisiertem Licht oder nicht, einer Fotografie, eines Scanners einer Synchrotron-Tomographie oder einer Röntgenstrahl-Bildgebung erfasst wird.

8. Erkennungsverfahren nach einem der vorhergehenden Ansprüche, wobei der automatische Lernalgorithmus (ALG) trainiert wird, um die Miniaturbilder der Lernbilder (IAP) durch Analyse der Farbe des zentralen Pixels der Miniaturbilder der segmentierten Bilder zu klassifizieren.

9. Erkennungsverfahren nach Anspruch 8, wobei das erfasste Bild der Gesteinsprobe (IER) in eine Vielzahl von Miniaturbildern zerlegt wird, indem für jedes Pixel des erfassten Bilds der Gesteinsprobe (IER) ein Zerlegen eines das Pixel umgebenden Miniaturbilds durchgeführt wird, und das vom automatischen Lernen (ALG) gebildete Modell (MOD) verwendet wird, um zu bestimmen, ob das Pixel zum geologischen Bestandteil (cge) gehört.

10. Erkennungsverfahren nach einem der Ansprüche 1 bis 7, wobei der automatische Lernalgorithmus (ALG) trainiert wird, um die ganze Fläche der Miniaturbilder der Lernbilder zu segmentieren, um darin die geologischen Bestandteile zu begrenzen.

11. Erkennungsverfahren nach Anspruch 10, wobei die geologischen Bestandteile (cge) erkannt werden, indem für jedes Miniaturbild des erfassten Bilds der Gesteinsprobe (IER) die geologischen Bestandteile begrenzt werden.

12. Erkennungsverfahren nach einem der vorhergehenden Ansprüche, wobei das Erkennungsverfahren mindestens einen zusätzlichen Schritt aufweist, der ausgewählt wird unter:
- Zählen der Anzahl von geologischen Bestandteilen (cge) des erfassten Bilds der Gesteinsprobe (IER) ausgehend von der Erkennung des geologischen Bestandteils, oder
- Bestimmen des von den geologischen Bestandteilen (cge) eingenommenen Anteils des Volumens der Gesteinsprobe ausgehend von der Erkennung des geologischen Bestandteils, oder
- Schätzen von Morphologie- und/oder Texturmerkmalen der geologischen Bestandteile (cge), oder
- Anwenden eines überwachten Klassifizierungsverfahrens, um die geologischen Bestandteile (cge) ab der Erkennung des geologischen Bestandteils zu kategorisieren.

13. Verfahren zur Nutzung eines Bodens oder Untergrunds, wobei die folgenden Schritte durchgeführt werden:
a) mindestens ein geologischer Bestandteil (cge) einer Gesteinsprobe wird mittels des Erkennungsverfahrens nach einem der vorhergehenden Ansprüche erkannt; und
b) der Boden oder der Untergrund wird abhängig von der Erkennung des geologischen Bestandteils (cge) des mindestens einen Gesteins genutzt.

14. Verfahren zur Nutzung eines Bodens oder eines Untergrunds nach Anspruch 13, wobei die Nutzung des Bodens oder des Untergrunds die Errichtung eines Bauwerks auf dem Boden oder in dem Untergrund, das Lagern von Gas im Untergrund, oder die Nutzung von Rohstoffen des Bodens oder des Untergrunds betrifft, wobei die Rohstoffe vorzugsweise das Gestein selbst oder ein im Boden oder im Untergrund enthaltenes Material oder Fluid sind.

15. Verfahren zur Bestimmung des Klimas in einer geographischen Zone über die geologischen Zeitalter hinweg, wobei die folgenden Schritte durchgeführt werden:
a) mindestens zwei Gesteinsproben werden in verschiedenen Tiefen einer unterirdischen Formation entnommen;
b) mindestens ein geologischer Bestandteil (cge) für jede Gesteinsprobe wird mittels des Erkennungsverfahrens nach einem der Ansprüche 1 bis 12 erkannt; und
c) das Klima sowie das geologische Zeitalter in der geographischen Zone werden abhängig von dem mindestens einen erkannten geologischen Bestandteil (cge) bestimmt.

## Claims

1. Method for detecting at least one geological constituent (cge) of a rock sample, from training images, **characterized in that** the following steps are carried out by means of a computing system:
a) segmenting (SEG) said training images (IAP) into at least two colours: a first colour for each area of said training images (IAP) comprising no geological constituent, and at least a second colour for each area of said training images (IAP) comprising a geological constituent (3);
b) partitioning (DEC) said training images (IAP) and said segmented images into a plurality of patches;
c) training a machine-learning classification algorithm (ALG) by means of said patches of said training images (IAP) and by means of said patches of said segmented training images so as to classify said patches of said training images (IAP) depending on said colour of said patches of said corresponding segmented training images;
d) acquiring an image of said rock sample (IER);
e) partitioning (DEC) said acquired image of said rock sample (IER) into a plurality of patches; and
f) detecting said at least one geological constituent (cge) in each of said patches of said acquired image of said rock sample (IER) by applying a model (MED) formed by said machine-learning algorithm (ALG) to said patches of said acquired image of said rock sample (IER).

2. Detecting method according to Claim 1, wherein said geological constituent (cge) is selected from among microfossils, nanofossils, plant debris, minerals, and pollen spores.

3. Detecting method according to either one of the preceding claims, wherein each one of said at least second colours corresponds to one type of geological constituent (cge).

4. Detecting method according to any one of the preceding claims, wherein the number of training images (IAP) is comprised between 3 and 20, and preferably between 3 and 10.

5. Detecting method according to any one of the preceding claims, wherein said machine-learning algorithm (ALG) uses an artificial neural network, preferably a convolutional neural network or a fully convolutional neural network.

6. Detecting method according to any one of the preceding claims, wherein said image of said rock sample (IER) is acquired from a thin section of said rock sample.

7. Detecting method according to any one of the preceding claims, wherein said image of said rock sample (IER) is acquired by means of an optical or electron microscope, using polarized light or not, of photography, of a scanner, of synchrotron tomography or of X-ray imaging.

8. Detecting method according to any one of the preceding claims, wherein said machine-learning algorithm (ALG) is trained to classify said patches of said training images (IAP), by analysing the colour of the central pixel of said patches of said segmented images.

9. Detecting method according to Claim 8, wherein said acquired image of said rock sample (IER) is partitioned into a plurality of patches by carrying out, for each pixel of said acquired image of said rock sample (IER), partitioning of a patch surrounding said pixel, and said model (MOD) formed by said machine learning (ALG) is used to determine whether said pixel belongs to said geological constituent (cge).

10. Detecting method according to any one of Claims 1 to 7, wherein said machine-learning algorithm (ALG) is trained to segment the entire area covered by said patches of said training images so as to delineate therein said geological constituents.

11. Detecting method according to Claim 10, wherein said geological constituents (cge) are detected by delineating for each patch of said acquired image of said rock sample (IER) said geological constituents.

12. Detecting method according to any one of the preceding claims, wherein said detecting method comprises at least one additional step selected from among:
- counting the number of geological constituents (cge) of said acquired image of said rock sample (IER) based on detection of said geological constituent, or
- determining the proportion of the rock-sample volume occupied by geological constituents (cge) based on detection of said geological constituent, or
- estimating morphological and/or textural characteristics of said geological constituents (cge), or
- applying a supervised classification method to categorize said geological constituents (cge) based on detection of said geological constituent.

13. Method for exploiting a surface or subsurface region, wherein the following steps are carried out:
a) detecting at least one geological constituent (cge) of a rock sample by means of the detecting method according to any one of the preceding claims; and
b) exploiting said surface or subsurface region depending on said detection of said geological constituent (cge) of said at least one rock.

14. Method for exploiting a surface or subsurface region according to Claim 13, wherein said exploitation of the surface or subsurface region concerns constructing a structure on said surface or subsurface region, storing gas in the subsurface region or extracting raw materials from said surface or subsurface region, said raw materials preferably being the rock itself, or a material or a fluid contained in said surface or subsurface region.

15. Method for determining the climate in a geographical area through the geologic ages, wherein the following steps are carried out:
a) taking at least two rock samples at various depths of an underground formation;
b) detecting at least one geological constituent (cge) for each rock sample by means of the detecting method according to any one of Claims 1 to 12; and
c) determining said climate and the geologic age in said geographical area depending on said at least one detected geological constituent (cge).
